# EUROPEAN PATENT SPECIFICATION

(11) **EP 3 446 753 B1**
(45) Date of publication and mention of the grant of the patent: **11.12.2019**
(21) Application number: 18178180.8
(22) Date of filing: 18.06.2018
(51) Int. Cl.: A61K 38/08, A61P 25/18

(54) **NEW THERAPEUTIC USES OF PEPTIDES OF THE ADIPOKINETIC HORMONE FAMILY**
NEUE THERAPEUTISCHE VERWENDUNGEN VON PEPTIDEN DER ADIPOKINETISCHEN HORMONFAMILIE
NOUVELLES UTILISATIONS THÉRAPEUTIQUES DE PEPTIDES DE LA FAMILLE DES HORMONES ADIPOKINÉTIQUES

(30) Priority: 22.08.2017 TR 201712479
(43) Date of publication of application: 27.02.2019
(73) Proprietor: Mutlu, Oguz, Besiktas/Istanbul (TR)
(72) Inventor: Mutlu, Oguz, Istanbul (TR); Vales, Karel, Klecany (CZ)
(74) Representative: Mutlu, Aydin

(56) References cited:
- WO-A1-2016/153453
- US-A1- 2012 108 510
- OGUZ MUTLU ET AL: "Effects of the adipokinetic hormone/red pigment-concentrating hormone (AKH/RPCH) family of peptides on MK-801-induced schizophrenia models", FUNDAMENTAL & CLINICAL PHARMACOLOGY., vol. 32, no. 6, 27 June 2018 (2018-06-27), pages 589-602, XP055541308, FR ISSN: 0767-3981, DOI: 10.1111/fcp.12386
- OGUZ MUTLU ET AL: "Effects of chronic administration of adipokinetic and hypertrehalosemic hormone on animal behavior, BDNF, and CREB expression in the hippocampus and neurogenesis in mice", FUNDAMENTAL & CLINICAL PHARMACOLOGY., vol. 30, no. 1, 23 February 2016 (2016-02-23), pages 4-13, XP055294814, FR ISSN: 0767-3981, DOI: 10.1111/fcp.12165

### SEQUENCE LISTING

<110> Oguz Mutlu
<120> Therapeutic Use of Adipokinetic Hormone/Red Pigment-Concentrating
   Hormone (AKH/RPCH) Family of Peptides
<130> 28-16524-A
<160> 3
<170> PatentIn version 3.5
<210> 1
   <211> 8
   <212> PRT
   <213> Anax Imperator Mauricianus
<400> 1
   Glu Val Asn Phe Ser Pro Ser Trp
   1 5
<210> 2
   <211> 8
   <212> PRT
   <213> Libellula Auripennis
<400> 2
   Glu Val Asn Phe Thr Pro Ser Trp
   1 5
<210> 3
   <211> 8
   <212> PRT
   <213> Phormia Terrae-Novae
<400> 3
   Glu Leu Thr Phe Ser Pro Asp Trp
   1 5

## Description

### Technical Field of the Invention

The present invention is related to a new use of adipokinetic hormone/red pigment-concentrating hormone (AKH/RPCH) family of peptides. The current invention particularly relates to adipokinetic hormone/red pigment-concentrating hormone (AKH/RPCH) family of peptides for use in the treatment of a disease selected from the group consisting of schizophrenia, psychosis, obsessive-compulsive disorder, attention deficiency hyperactivity syndrome, mania and bipolar disorder.

### Background of the Invention

It is possible to find detailed information about the presence of peptides in insects especially about insect neuropeptides in literature. Concerning the peptides present in the insects, a categorization such as the following can be made: peptides involved in homeostasis and metabolism, peptides regulating reproduction, growth and development and peptides modifying spontaneous muscle contractions. A great variety of processes including metabolic, behavioural, developmental or reproductive in character in insects is known to be influenced or regulated by neuropeptides.

One of the major neuropeptide groups is the adipokinetic and hypertrehalosaemic peptides which belong to the AKH/RPCH family of peptides. Adipokinetic or hyperlipeamic effect is the increase of haemolymph lipid (diacylglycerol) concentration. Hypertrehalosaemic effect is the increase of haemolymph carbohydrates (sugar trehalose). Adipokinetic and hypertrehalosaemic peptides control fat, carbohydrate and protein metabolism. About 30 different peptides are known at present which renders this family as one of the largest family of peptides. All members are from 8 to 10 amino acids long, N-terminally blocked by a pyroglutamate residue and C- terminally blocked by an amide. At position 4 (Phe or Tyr) and 8 (Trp) aromatic residues are present; most variations in constituent amino acids are conservative.

Besides the hyperlipaemic and hypertrehalosaemic effects, the following are indicated as the major activities of peptides of the AKH/RPCH family.
- Stimulation of the frequency of the heart beat in Periplaneta Americana
- Increase in muscle tone and frequency of contraction of the spontaneous activity of the isolated leg of a locust
- Inhibition of protein synthesis in L. migratoria and in cricket Acheta domesticus
- Inhibition of fatty acid synthesis in S. gregaria
- Inhibition of RNA synthesis in fat body of L. migratoria

Adipokinetic hormones and hypertrehalosaemic hormones of insects comprise a family of peptide hormones that primarily regulate the levels of energy metabolites, such as trehalose (disaccharide), diacylglycerol and proline that circulate in the haemolymph. These peptide hormones are products of neurosecretory neurons located in the corpora cardiaca, neuroendocrine glands attached to the brain. The structural organization of the insect corpora cardiaca is similar to the hypothalamus-neurohypophysis of the vertebrate endocrine system *(Gade, G., Heather G., Adipokinetic and hypertrehalosaemic neurohormones in "Marco Biomedical and Life Sciences Encyclopaedia of Entomology", Springer reference).*

Adipokinetic hormones (AKHs) are metabolic neuropeptides, mediating mobilization of energy substrates from the fat body in many insects. Studies using transgenic manipulations of the dAkh gene have demonstrated that AKH induces both hypertrehalosaemia and hyperlipaemia. Moreover, it is known that AKH peptides have excitatory effects on motor neurons.

Using a heterologous (in locusts and cockroaches) and a homologous bioassay, the neuropeptide pGlu-Val-Asn-Phe-Ser-Pro-Ser-Trp-NH₂ was isolated from extracts of corpora cardiaca of the Emperor dragonfly, *Anax imperator.* Low concentrations of the synthetic peptide injected into the Emperor dragonfly increased the haemolymph lipid concentration, suggesting a possible role of the peptide in lipid homeostasis during flight. Therefore, it is named Ani-AKH, *Anax imperator* adipokinetic hormone *(*Gade G, Janssens MP, Kellner R. A novel peptide in the AKH/RPCH family isolated from the corpora cardiaca of the Emperor dragonfly, Anax imperator. Peptides, Vol. 15, No. 1, pp. 1-6, 1994*)*.

In a previous study, it is suggested that adipokinetic hormone may contribute to the neuronal function in the human central nervous system. This study reveals that an antiserum raised against locust adipokinetic hormone I can display a considerable quantity of adipokinetic hormone-like immunoreactivity in the human cerebrospinal fluid. As a result of the gel permeation and high performance liquid chromatography, the main immunoreactive component in the cerebrospinal fluid coeluted with adipokinetic hormone I. These results suggest that adipokinetic hormone may contribute to the neuronal function in the human central nervous system *(*Suzuki H, Sato S, Tsuchiya T, Suzuki Y, Muramatsu H, Ishihara N, Shimoda S. Identification and characterization of adipokinetic hormone (Locusta migratoria)-like immunoreactivity in the human cerebrospinal fluid, Biochemical and biophysical research communications; 1989; 163 (1): 534-540*).*

In another study, it has been identified the first insect AKH receptors, namely those from the fruit fly *Drosophila melanogaster* and the silkworm *Bombyx mori.* The results of this study represent a breakthrough for insect molecular endocrinology, because it will lead to the cloning of all AKH receptors from all model insects used in AKH research, and, therefore, to a better understanding of AKH heterogeneity and actions. It is expressed in this study that the insect AKH receptors are structurally and evolutionarily related to the gonadotropin-releasing hormone receptors from vertebrates *(*Staubli et al., Molecular identification of the insect adipokinetic hormone receptors, Proc. Natl. Acad. Sci. USA. 2002 Mar 19;99(6):3446-51*.).*

In a study performed in rat, a novel peptidergic system was identified in the rat central nervous system by using an antiserum to locust adipokinetic hormone I. Immunoreactive fibers were present in the hypothalamic median eminence and periventricular nucleus and the spinal cord dorsal horn, intermediolateral cell column and sacral parasympathetic nucleus. Immunoreactive cells were present in the dorsal gray commissure of lumbosacral spinal cord, the hypothalamic periventricular nucleus and cerebral cortex. *(*Sasek CA, Schueler PA, Herman WS, Elde RP. An antiserum to locust adipokinetic hormone reveals a novel peptidergic system in the rat central nervous system. Brain Res. 1985 Sep 16; 343(1):172-5*).*

There are many studies in the prior art directed more particularly to the AKH family as mentioned above. In one other of these studies, it is determined that AKHs respond to stress situations including oxidative stress and insecticide treatment when applied to the insects elicit an intensive AKH increase in haemolymph. It is also observed in the same study that when AKH is applied to insects by mixing with insecticides, it is observed that the carbon dioxide production and the metabolic rate are increased thus bug mortality is also increased. It is thought that this enhanced effect of the insecticides probably resulted from the stimulatory role of AKH on bug metabolism *(*Velki M., Kodrík D., Večeřa J., Hackenberger B.K., Socha R. Oxidative stress elicited by insecticides: a role for the adipokinetic hormone. Gen. Comp. Endocrinol. 2011; 172(1): 77-84*).*

The functions of adipokinetic hormones in lipid metabolism are also subject to many patent applications as well. For instance, US 6,852,693 B2 is related to a method for using polypeptide compounds based on the structures of insect peptides of the adipokinetic hormone family to mobilize lipids in humans. Said application discloses these compositions and peptides could be useful for modulating human body weight, such as inducing weight loss and methods for identifying other compounds effective for modulating lipid mobilization in human.

In addition to these, in a recent study of the inventor, it has been shown that (AKH/RPCH) peptide family can be used in depression, anxiety, pain and locomotor disorders, and exert neuroprotective effects *(*Mutlu O, Gumuslu E, Kokturk S, Ulak G, Akar F, Erden F, Kaya H, Tanyeri P. Effects of chronic administration of adipokinetic and hypertrehalosemic hormone on animal behavior, BDNF and CREB expression in the hippocampus and neurogenesis in mice. Fundam Clin Pharmacol. 2016; 30(1): 4-13*).*

For many years, positive psychotic symptoms were the primary target in the treatment of schizophrenia. However, schizophrenia is also associated with cognitive dysfunction (Green et al., 2000). Cognitive impairment in schizophrenia is common, and it decreases the quality and function of life (Addington and Addington, 1999); these impairments manifest primarily as disruptions in working memory as well as learning and attention. Cognitive dysfunctions indicate the initial illness and lead to the development of psychotic symptoms (Tollefson, 1996). These problems also diminish the ability of the patient to care for oneself and increase the frequency of hospitalization, thus increasing the cost to society (Sevy and Davidson, 1995). Therefore, effective treatment of cognitive deficits in schizophrenic patients may greatly impact the patient's quality of life.

N-methyl-D-aspartate (NMDA) receptor is a subclass of ionotropic glutamate receptors. Antagonists of the NMDA receptor block hippocampal long-term potentiation and impair hippocampal-dependent behavior (e.g., spatial memory tasks) (Bischoff and Tiedtke, 1992). Non-competitive antagonists of the NMDA receptor, such as ketamine or phencyclidine (PCP), have strong psychotomimetic effects in humans (Javitt and Zukin, 1991). MK-801 is a non-competitive antagonist that binds to the PCP binding site within the NMDA receptor-ion complex (Wong and Nielsen, 1989). It impairs animal performance in various learning and memory paradigms (Castellano et al., 2001 and Riedel et al., 2003). MK-801 also produces various effects on rodent behavior, including deficits in sensory processing (Al-Amin and Schwarzkopf, 1996), hypermotility (Carlsson, 1993), stereotypy and ataxia (Tricklebank et al., 1989).

MK-801 is the most frequently used non-competitive NMDA receptor antagonist in animal models to mimic psychosis for experimental purposes. MK-801 blocks NMDA receptors in a use- and voltage-dependent manner, since the channel must open for the drug to bind inside it. MK-801 is also associated with a number of negative side effects, including cognitive disruption and psychotic-spectrum reactions. It also inhibited the induction of long term potentiation. MK-801 has a great deal of potential to be used in research in creating animal models of schizophrenia. Unlike dopaminergic agonists, which mimic only the positive symptoms of schizophrenia, a single injection of MK-801 was successful in modelling both the positive and negative symptoms of schizophrenia.

Prepulse Inhibition (PPI) is a neurological phenomenon in which a weaker prestimulus (prepulse) inhibits the reaction of an organism to a subsequent strong startling stimulus (pulse). Disruptions of PPI are studied in humans and many other species. The most studied are deficits of PPI in schizophrenia, although this disease is not the only one to be associated with such deficits. MK-801 disturbs PPI (Bast et al. 2000) and can be used as a pharmacological model of schizophrenia (Eyjolfsson et al. 2006), psychosis and attention deficiency hyperactivity disorder (Levin et al. 2011). A lot of studies have shown that PPI is disturbed in many diseases and disorders, for example in schizophrenia *[*S. G. Ewing and A. A. Grace, "Evidence for impaired sound intensity processing during prepulse inhibition of the startle response in a rodent developmental disruption model of schizophrenia, "Journal ofPsychiatric Research, vol.47, no.11, pp.1630-1635, 2013*],* obsessive-compulsive disorder (Swerdlow et al., 1993) and attention deficiency hyperactivity disorder (Ornitz et al. 1992). MK-801 and amphetamine induced hyperactivity can be also used as a model of mania and manic episode of bipolar disorder (Hodes et al. 2015).

As mentioned above, several properties of the AKHs of the large peptide family were determined with the studies in the prior art. The studies are still going on nowadays. Studies are also going on for the illnesses of schizophrenia, psychosis, obsessive-compulsive disorder, attention deficiency hyperactivity syndrome, mania and bipolar disorder. The current invention relates to adipokinetic hormone/red pigment-concentrating hormone (AKH/RPCH) family of peptides for use in the treatment of these illnesses as alternative and functional agents.

### Brief Disclosure of the Invention

The present invention relates to a new use of a peptide of the adipokinetic hormone/red pigment-concentrating hormone (AKH/RPCH) family of peptides. It is particularly related to the use of said peptide in the treatment of a disease selected from the group consisting of schizophrenia, psychosis, obsessive-compulsive disorder, attention deficiency hyperactivity syndromes, mania and bipolar disorder or a combination thereof.

The peptide hormones of the invention have the chemical structure shown below:
**Xaa¹ - Xaa² - Xaa³ - Xaa⁴ - Xaa⁵ - Xaa⁶ - Xaa⁷ - Xaa⁸ - X - NH₂**
wherein;
Xaa¹ is a pyroglutamate residue,
Xaa² residue is selected from a group consisting of Leu, Val, Ile, Phe, Tyr amino acids,
Xaa³ residue is selected from one of Asn, Thr amino acids,
Xaa⁴ residue is selected from one of Phe, Tyr amino acids,
Xaa⁵ residue is selected from one of Thr, Ser amino acids,
Xaa⁶ residue is selected from one of Pro, Ser, Thr, Ala amino acids,
Xaa⁷ residue is selected from one of Asn, Gly, Ser, Asp, Trp, Val amino acids,
Xaa⁸ residue is selected as Trp amino acid,
X is a residue having 0 to 2 amino acid residues, it is preferably 0; in case there are 1-2 amino acids, it is Gly residue for the first and one of Gly, Thr, Asn, Ser, Tyr, Trp residues for the second.

In a preferred embodiment of the invention the peptide hormones of the invention has the following sequence:
**pGlu-Val-Asn-Phe-Xaa⁵-Pro-Ser-Trp-NH₂**
wherein Xaa⁵ residue is selected from Ser and Thr residues.

In another aspect, the peptide hormone of the invention has the following sequence:
**pGlu-Leu-Thr-Phe-Ser-Pro-Asp-Trp-NH₂**

In the embodiments of the invention, the disease in which the peptide hormone related to the invention is effective, is selected from the group consisting of schizophrenia, psychosis, obsessive-compulsive disorder, attention deficiency hyperactivity syndrome, mania and bipolar disorder or a combination thereof.

The invention, in another aspect is about a pharmaceutical composition comprising the peptide hormone mentioned above and at least one excipient.

The invention, in another aspect is related to the peptide hormone mentioned above for use in treatment of hyperactivity disorder manifested by a disease selected from the group consisting of attention deficiency hyperactivity syndrome, mania and bipolar disorder.

### Brief Description of the Figures

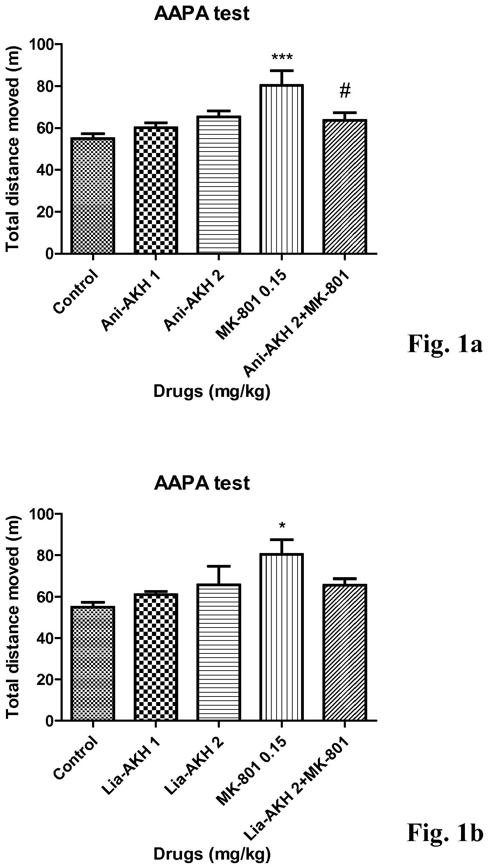

**Figure 1a** illustrates the effect on total distance moved (m) obtained by administering intraperitoneally (i.p.) Ani-AKH *(Anax Imperator Mauricianus* AKH) (1 and 2 mg/kg), MK-801 (0.15 mg/kg), Ani 2+MK-801 for 4 days in the Active Allothetic Place Avoidance test (AAPA) in Long-Ewans rats. Data is indicated as ± standard error of the mean. (***p<0.001 compared to the control group, #p<0.05 compared to the MK-801 alone group).
**Figure 1b** illustrates the effect on total distance moved (m) obtained by administering intraperitoneally (i.p.) Lia-AKH (*Libellula Auripennis* AKH) (1 and 2 mg/kg), MK-801 (0.15 mg/kg), Lia 2+MK-801 for 4 days in the Active Allothetic Place Avoidance test (AAPA) in Long-Ewans rats. Data is indicated as ± standard error of the mean (*p<0.05 compared to the control group).

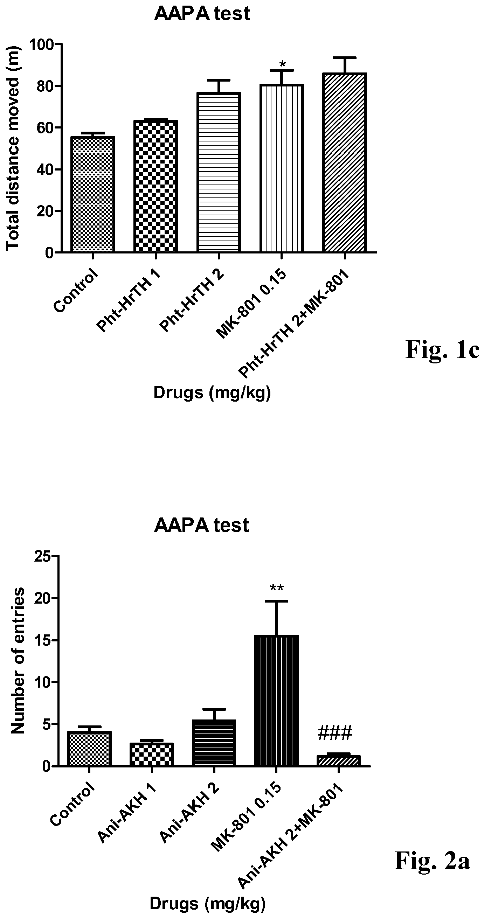

**Figure 1c** illustrates the effect on total distance moved (m) obtained by administering intraperitoneally (i.p.) Pht-HrTH (*Phormia-Terrae* hypertrehalosaemic hormone (HrTH)) (1 and 2 mg/kg), MK-801 (0.15 mg/kg), Pht-HrTH 2+MK-801 for 4 days in the Active Allothetic Place Avoidance test (AAPA) in Long-Ewans rats. Data is indicated as ± standard error of the mean (*p<0.05 compared to the control group).
**Figure 2a** illustrates the effect on number of entries obtained by administering intraperitoneally (i.p.) Ani-AKH *(Anax Imperator Mauricianus* AKH) (1 and 2 mg/kg), MK-801 (0.15 mg/kg), Ani 2+MK-801 for 4 days in the Active Allothetic Place Avoidance test (AAPA) in Long-Ewans rats. Data is indicated as ± standard error of the mean (**p<0.01 compared to the control group, ###p<0.001 compared to the MK-801 alone group).

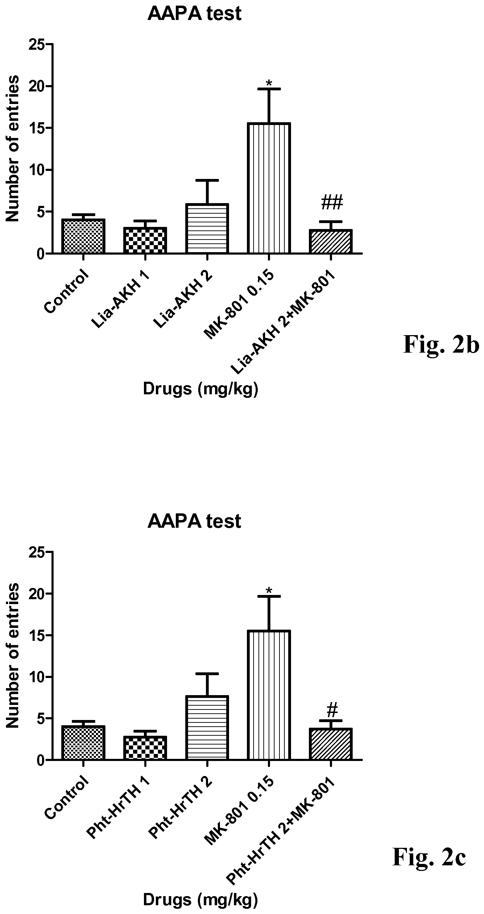

**Figure 2b** illustrates the effect on number of entries obtained by administering intraperitoneally (i.p.) Lia-AKH (*Libellula Auripennis* AKH) (1 and 2 mg/kg), MK-801 (0.15 mg/kg), Lia 2+MK-801 for 4 days in the Active Allothetic Place Avoidance test (AAPA) in Long-Ewans rats. Data is indicated as ± standard error of the mean (*p<0.05 compared to the control group, ##p<0.01 compared to the MK-801 alone group).
**Figure 2c** illustrates the effect on number of entries obtained by administering intraperitoneally (i.p.) Pht-HrTH (*Phormia-Terrae* HrTH) (1 and 2 mg/kg), MK-801 (0.15 mg/kg), Pht-HrTH 2+MK-801 for 4 days in the Active Allothetic Place Avoidance test (AAPA) in Long-Ewans rats. Data is indicated as ± standard error of the mean (*p<0.05 compared to the control group, #p<0.05 compared to the MK-801 alone group).

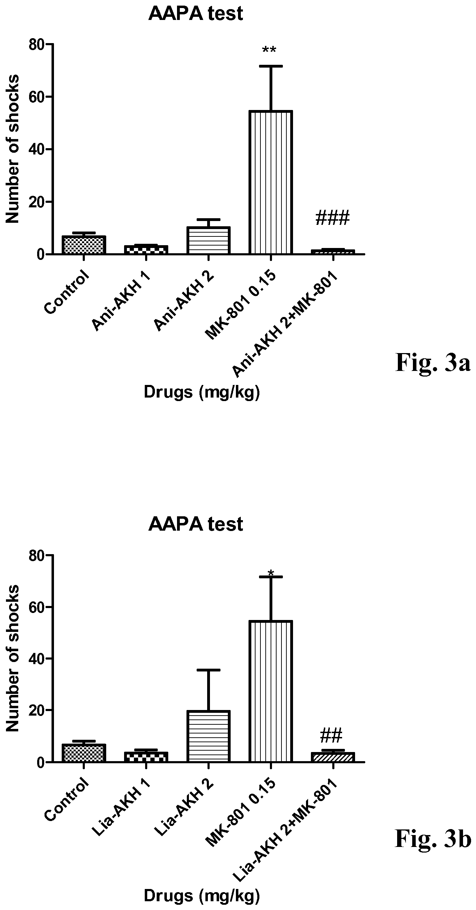

**Figure 3a** illustrates the effect on number of shocks obtained by administering intraperitoneally (i.p.) Ani-AKH (*Anax Imperator Mauricianus* AKH) (1 and 2 mg/kg), MK-801 (0.15 mg/kg), Ani 2+MK-801 for 4 days in the Active Allothetic Place Avoidance test (AAPA) in Long-Ewans rats. Data is indicated as ± standard error of the mean (**p<0.01 compared to the control group, ###p<0.001 compared to the MK-801 alone group).
**Figure 3b** illustrates the effect on number of shocks obtained by administering intraperitoneally (i.p.) Lia-AKH (*Libellula Auripennis* AKH) (1 and 2 mg/kg), MK-801 (0.15 mg/kg), Lia 2+MK-801 for 4 days in the Active Allothetic Place Avoidance test (AAPA) in Long-Ewans rats. Data is indicated as ± standard error of the mean (*p<0.05 compared to the control group, ##p<0.01 compared to the MK-801 alone group).

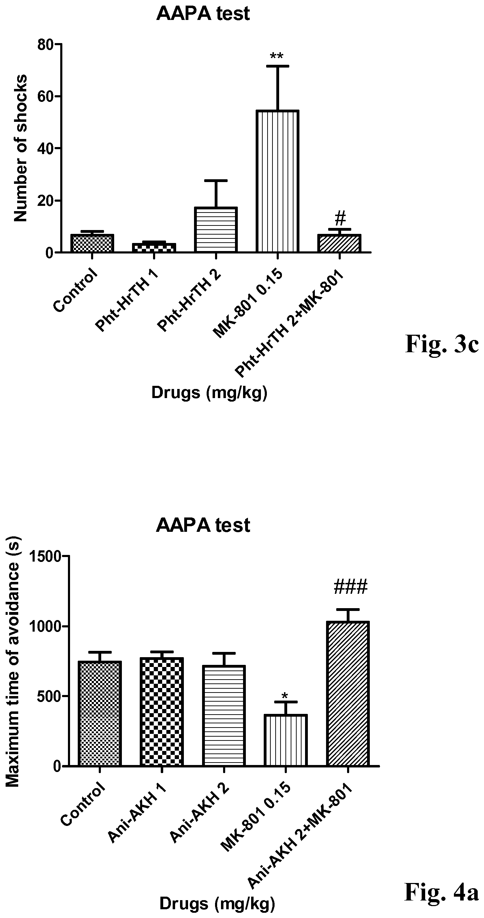

**Figure 3c** illustrates the effect on number of shocks obtained by administering intraperitoneally (i.p.) Pht-HrTH (*Phormia-Terrae* HrTH) (1 and 2 mg/kg), MK-801 (0.15 mg/kg), Pht-HrTH 2+MK-801 for 4 days in the Active Allothetic Place Avoidance test (AAPA) in Long-Ewans rats. Data is indicated as ± standard error of the mean (**p<0.01 compared to the control group, #p<0.05 compared to the MK-801 alone group).
**Figure 4a** illustrates the effect on maximum time of avoidance (s) obtained by administering intraperitoneally (i.p.) Ani-AKH (*Anax Imperator Mauricianus* AKH) (1 and 2 mg/kg), MK-801 (0.15 mg/kg), Ani 2+MK-801 for 4 days in the Active Allothetic Place Avoidance test (AAPA) in Long-Ewans rats. Data is indicated as ± standard error of the mean (*p<0.05 compared to the control group, ###p<0.001 compared to the MK-801 alone group).

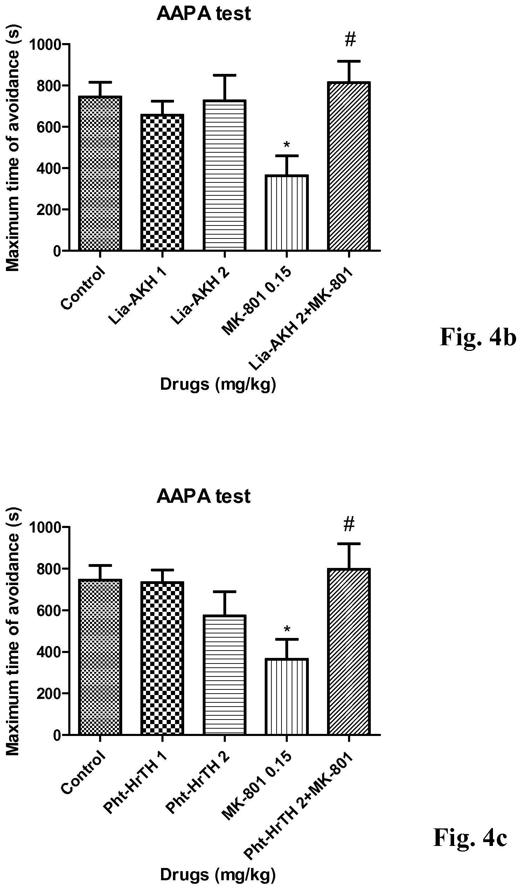

**Figure 4b** illustrates the effect on maximum time of avoidance (s) obtained by administering intraperitoneally (i.p.) Lia-AKH (*Libellula Auripennis* AKH) (1 and 2 mg/kg), MK-801 (0.15 mg/kg), Lia 2+MK-801 for 4 days in the Active Allothetic Place Avoidance test (AAPA) in Long-Ewans rats. Data is indicated as ± standard error of the mean (*p<0.05 compared to the control group, #p<0.05 compared to the MK-801 alone group).
**Figure 4c** illustrates the effect on maximum time of avoidance (s) obtained by administering intraperitoneally (i.p.) Pht-HrTH (*Phormia-Terrae* HrTH) (1 and 2 mg/kg), MK-801 (0.15 mg/kg), Pht-HrTH 2+MK-801 for 4 days in the Active Allothetic Place Avoidance test (AAPA) in Long-Ewans rats. Data is indicated as ± standard error of the mean (*p<0.05 compared to the control group, #p<0.05 compared to the MK-801 alone group).

### Detailed Description of the Invention

The present invention is related to a new use of adipokinetic hormone/red pigment-concentrating hormone (AKH/RPCH) family of peptides in treatment of a disease selected from the group consisting of schizophrenia, psychosis, obsessive-compulsive disorder, attention deficiency hyperactivity syndrome, mania and bipolar disorder or a combination thereof.

The term "peptide" refers to an amino acid (aa) polymer of adipokinetic hormone/red pigment-concentrating hormone (AKH/RPCH) family. Said peptide signifies preferably a peptide from 8 to 10 amino acids long, N-terminally blocked by a pyroglutamate residue and C- terminally blocked by an amide. More particularly, said peptide refers to a peptide having aromatic residues at position 4 (Phe or Tyr) and 8 (Trp).

In a further aspect, the present invention relates to pharmaceutical compositions comprising the peptides mentioned above which prevents, ameliorates or cures said diseases.
The invention is particularly related to the peptide hormone with the following chemical structure for use in treatment of a disease selected from the group consisting of schizophrenia, psychosis, obsessive-compulsive disorder, attention deficiency hyperactivity syndrome, mania and bipolar disorder;
**Xaa¹** - **Xaa²** - **Xaa³** - **Xaa⁴** - **Xaa⁵** - **Xaa⁶** - **Xaa⁷** - **Xaa⁸** - **X** - **NH₂**
wherein;
Xaa¹ is a pyroglutamate residue,
Xaa² residue is selected from a group consisting of Leu, Val, Ile, Phe, Tyr amino acids,
Xaa³ residue is selected from one of Asn, Thr amino acids,
Xaa⁴ residue is selected from one of Phe, Tyr amino acids,
Xaa⁵ residue is selected from one of Thr, Ser amino acids,
Xaa⁶ residue is selected from one of Pro, Ser, Thr, Ala amino acids,
Xaa⁷ residue is selected from one of Asn, Gly, Ser, Asp, Trp, Val amino acids,
Xaa⁸ residue is selected as Trp amino acid,

X is a residue having 0 to 2 amino acid residues, it is preferably 0; in case there are 1-2 amino acids, it is Gly residue for the first and one of Gly, Thr, Asn, Ser, Tyr, Trp residues for the second.

In a preferred embodiment of the invention, the amino acid sequence of the peptide hormones of the invention is as follows:
**pGlu-Val-Asn-Phe-Xaa⁵-Pro-Ser-Trp-NH₂**
wherein Xaa⁵ residue is selected among Ser and Thr amino acids.

In another aspect, the peptide hormone of the invention has the following amino acid sequence:
**pGlu-Leu-Thr-Phe-Ser-Pro-Asp-Trp-NH₂**
In another aspect, the present invention relates to a pharmaceutical composition comprising a peptide of the adipokinetic hormone/red pigment-concentrating hormone peptide family (AKH/RPCH) as active agent and at least one pharmaceutically acceptable excipient. Said pharmaceutical composition is suitable for use in human.

The invention, in another aspect is related to the peptide hormone mentioned above for use in treatment of hyperactivity disorder manifested by a disease selected from the group consisting of attention deficiency hyperactivity syndrome, mania and bipolar disorder.

The study using a rat model related to the adipokinetic hormone/red pigment-concentrating hormone peptide family is presented below. The peptides used in this study are presented in Table 1 with their code names and amino acid sequences.

**Table 1- Adipokinetic hormone/red pigment-concentrating hormone peptides used in rat model (AKH/RPCH)**

| **AKH/RPCH Family - Code Name** | **CAS No** | **Amino acid Sequence - Chemical Name** |
|---|---|---|
| Ani-AKH | 154512-22-8 | pGlu-VNFSPSW-NH2 |
| (Anax Imperator Mauricianus) | | pGlu-Val-Asn-Phe-Ser-Pro-Ser-Trp-NH2 *5-oxo-L-prolyl-L-valyl-L-asparaginyl-L-phenylalanyl-L-seryl-L-prolyl-L-seryl-L-Tryptophanamide* |
| Lia-AKH | 129536-34-1 | PGlu-VNFTPSW-NH2 |
| (Libellula Auripennis) | | pGlu-Val-Asn-Phe- Thr-Pro-Ser- Trp-NH2 *5-oxo-L-prolyl-L-valyl-L-asparaginyl-L-phenylalanyl-L-threonyl-L-prolyl-L-seryl-L-Tryptophanamide* |
| Pht-HrTH | 129204-82-6 | pGlu-LTFSPDW-NH2 |
| Hypertrehalosaemic hormone | | pGlu-Leu-Thr-Phe-Ser-Pro-Asp-Trp-NH2 *5-oxo-L-prolyl-L-leucyl-L-threonyl-L-phenylalanyl-L-seryl-L-prolyl-L-alfa-aspartyl-L-Tryptophanamide* |
| (Phormia Terrae-Novae) | | |

### Examples

### The study of adipokinetic hormone/red pigment-concentrating hormone peptides in rat models

### 1. Animals

3-4 month-old male rats of the Long Evans strain were used for the Active Allothetic Place Avoidance test and were obtained from the accredited breeding colony of the Institute of Physiology, Academy of Science, Prague. Rats were acclimatised for 7-10 days prior to testing, during which they were weighed twice and handled four times. Rats were housed in pairs in transparent plastic cages (30×30×40 cm) in an air-conditioned animal room with a constant temperature of 21 °C and with 12/12 light/dark cycle (lights on at 7:00). All animals received food and water ad libitum. All experiments respected the Guidelines of the European Union (86/609/EU) and the National Committee for the Care and Use of Laboratory Animals (Czech Republic) and were approved by the committee under the number: MEYSCR-27527/2012-31.

### 2. Experimental groups and drug administration

In the Active Allothetic Place Avoidance test, Long Evans rats were treated with Ani-AKH (1 and 2 mg/kg) (n=8), Lia-AKH (1 and 2 mg/kg) (n=8), Pht-HrTH (1 and 2 mg/kg) (n=8), MK-801 (0.15 mg/kg) (n=8), Ani-AKH (2 mg/kg)+MK-801 (0.15 mg/kg) (n=8), Lia-AKH (2 mg/kg)+MK-801 (0.15 mg/kg) (n=8), Pht-HrTH (2 mg/kg)+MK-801 (0.15 mg/kg) (n=8) or vehicle (control group=saline with 15% DMSO) (n= 8) for 4 days (subchronically). Ani-AKH, Lia-AKH and Pht-HrTH were administered intraperitoneally (i.p.) 60 min. before the Active Allothetic Place Avoidance test while MK-801 was administered (i.p.) 40 min. before the test in a volume of 2ml/kg body weight. Separate group of animals were used in each test. The doses of the drugs were chosen according to the previous studies (Mutlu et al. 2016).

### 3. Drugs

(+)-MK-801 maleate ((5S,10R)-(+)-5-methyl-10,11-dihydro-5H-dibenzo(a, d)-cyclo-hepten-5,10-imine maleate) was obtained from Tocris Bioscience, United Kingdom. Anax imperator AKH (Ani-AKH), Libellula auripennis AKH (Lia-AKH) and Phormia-Terra hypertrehalosemic hormone (Pht-HrTH) were purchased from TRC (Toronto/Canada). MK-801 was dissolved in saline while AKH was dissolved in saline mixed with 15% DMSO. Saline with 15% DMSO was used as control group.

### 4. Active Allothetic Place Avoidance Test (AAPA)

The Active Allothetic Place Avoidance Test is a spatial memory test that can determine the ability of animals to organize their behavior efficiently in both normal and pathological conditions. Successful performance in the test requires the rat to solve a conflict between two discordant subsets of spatial stimuli (extramaze and intramaze cues). It is believed that successful performance in the Active Allothetic Place Avoidance Test, where the rat has to differentiate between relevant and irrelevant stimuli, depends on the mode of information processing disturbed in schizophrenic patients (Bubenikova-Valesova et al., 2008b). The Active Allothetic Place Avoidance arena was described in detail in previous papers (Cimadevilla et al., 2001). Briefly, it consisted of a smooth metallic circular arena (82 cm in diameter), enclosed by a 30 cm high transparent Plexiglas wall. The arena was elevated 1 m above the floor of 4×5 m experimental room containing an abundance of extramaze landmarks. The arena together with the wall was rotated (1 rpm) by an electric motor placed underneath the arena disc. At the beginning of each training session, a rat was placed in the arena at a location opposite an imperceptible 60° to-be- avoided sector (shock sector), which was defined by the computer- based tracking system (iTrack, Biosignal Group, USA), located in an adjacent room. The arena rotation started immediately after placement of the rat. The location of the shock sector could be determined solely by its spatial relation to the distal orienting cues located in the room. Rats were wearing a light latex harness, to which an infrared light-emitting diode (LED) was attached between the rat's shoulders. Whenever the rat entered the to-be-avoided sector for more than 500 ms (millisecond), the tracking system delivered a mild, constant-current shock (50 Hz, 0.5 s, 0.4-0.7 mA) and counted an entrance. If the rat did not leave the sector, additional shocks were given every 1200 ms, but no more entrances were counted until the rat left the sector for more than 300 ms. This procedure using shocks has previously been shown to be effective and safe for animals, leading to rapid avoidance behavior (Cimadevilla et al., 2001). The appropriate shock current (ranging between 0.4 and 0.7 mA) was carefully adjusted for each rat to evoke a rapid escape reaction but prevent freezing. Most animals responded appropriately to a shock intensity of 0.5 mA. The arena surface was carefully cleaned by a detergent solution between rats. For each of the groups, the same control and MK-801 treated rats were used as a reference. Animals were trained in four consecutive daily sessions in the Active Allothetic Place Avoidance Test. Experimental sessions in Active Allothetic Place Avoidance Test lasted 20 min, and each rat had one session every day, carried out during daylight hours.

### 5. Statistical Evaluation

The results of the AAPA test were evaluated by one-way ANOVA followed by Tukey's post-hoc test when significant differences were detected. The data were expressed as the mean values ± SEM. The differences were considered to be statistically significant when p was less than 0.05.

In the AAPA test, one-way ANOVA revealed significant differences for Ani-AKH, Lia-AKH and Pht-HrTH groups when the locomotor activity (total distance (m) moved) of the groups were compared [F(4,35)=5.50; p=0.0015; F(4,34)=3.40; p=0.019; F(4,34)=5.33, p=0.0019; respectively].

AKH treatment did not affect locomotion in naive rats. MK-801 significantly increased locomotion (p<0.001) compared to control and this effect was reversed by Ani-AKH 2 mg/kg treatment (p<0.05). Lia-AKH 2 mg/kg had a partial effect while Pht-HrTH 2 mg/kg had no reversing effect on locomotion.

In the AAPA test, one-way ANOVA revealed significant differences for Ani-AKH, Lia-AKH and Pht-HrTH groups when the number of entries of the groups to the shock sector were compared [F(4,35)=8.09; p<0.0001; F(4,34)=5.33; p=0.0019; F(4,34)=4.98, p=0.0029; respectively]. MK-801 treatment significantly increased number of entries (p<0.01) while Ani-AKH 2 mg/kg (p<0.001), Lia-AKH 2 mg/kg (p<0.01) and Pht-HrTH 2 mg/kg (p<0.05) significantly reversed this effect.

In the AAPA test, one-way ANOVA revealed significant differences for Ani-AKH, Lia-AKH and Pht-HrTH groups when the exposed number of shocks of the groups were compared [F(4,35)=7.95; p=0.0001; F(4,34)=4.61; p=0.0044; F(4,34)=5.22, p=0.0022; respectively]. MK-801 treatment significantly increased number of shocks (p<0.01) while Ani-AKH 2 mg/kg (p<0.001), Lia-AKH 2 mg/kg (p<0.01) and Pht-HrTH 2 mg/kg (p<0.05) significantly reversed this effect.

In the AAPA test, one-way ANOVA revealed significant differences for Ani-AKH, Lia-AKH and Pht-HrTH groups when the maximum time of avoidance (s) of the groups were compared [F(4,35)=8.60; p<0.0001; F(4,34)=3.62; p=0.014; F(4,34)=3.50, p=0.0169; respectively]. MK-801 treatment significantly decreased maximum time of avoidance (p<0.05) while Ani-AKH 2 mg/kg (p<0.001), Lia-AKH 2 mg/kg (p<0.05) and Pht-HrTH 2 mg/kg (p<0.05) significantly reversed this effect.

### 6. Results

Ani-AKH (2 mg/kg), Lia-AKH (2 mg/kg) and Pht-HrTH (2 mg/kg) when administered subchronically significantly has been observed to reverse MK-801 (0.15 mg/kg) induced cognitive memory impairment effects in the Active Allothetic Place Avoidance Test. MK-801 induced increased locomotion was significantly reversed by Ani-AKH (2 mg/kg) treatment while Lia-AKH (2 mg/kg) had a partial effect. According to these results, AKH/RPCH family peptides may show activity in the treatment of schizophrenia, psychosis, obsessive-compulsive disorder, attention deficiency hyperactivity syndrome, mania and bipolar disorder.

## Claims

1. A peptide hormone of the AKH/RPCH peptide family for use in treatment of a disease selected from schizophrenia, psychosis, obsessive-compulsive disorder, attention deficiency hyperactivity syndrome, mania and bipolar disorder; wherein said peptide has the following amino acid sequence:
**Xaa¹** - **Xaa²** - **Xaa³** - **Xaa⁴** - **Xaa⁵** - **Xaa⁶** - **Xaa⁷** - **Xaa⁸** - **X** - **NH₂**
wherein;
Xaa1 is a pyroglutamate residue,
Xaa² residue is selected from a group consisting of Leu, Val, Ile, Phe, Tyr amino acids,
Xaa³ residue is selected from one of Asn, Thr amino acids,
Xaa⁴ residue is selected from one of Phe, Tyr amino acids,
Xaa⁵ residue is selected from one of Thr, Ser amino acids,
Xaa⁶ residue is selected from one of Pro, Ser, Thr, Ala amino acids,
Xaa⁷ residue is selected from one of Asn, Gly, Ser, Asp, Trp, Val amino acids,
Xaa⁸ residue is selected as Trp amino acid,
and X residue is between the 0 to 2 amino acid residue; it is preferably 0, in case there are 1-2 amino acids, it is Gly residue for the first and one of Gly, Thr, Asn, Ser, Tyr, Trp residues for the second.

2. A peptide hormone for use according to claim 1, wherein said peptide hormone has the following amino acid sequence:
**pGlu-Val-Asn-Phe-Xaa⁵-Pro-Ser-Trp-NH₂**
wherein; Xaa⁵ residue is selected from Ser and Thr amino acids.

3. A peptide hormone for use according to claim 1, wherein said peptide hormone has the following amino acid sequence:
**pGlu-Leu-Thr-Phe-Ser-Pro-Asp-Trp-NH₂**

4. A peptide hormone for use according to any of the claims 1 to 3, wherein said disease is schizophrenia.

5. A peptide hormone for use according to any of the claims 1 to 3, wherein said disease is psychosis.

6. A peptide hormone for use according to any of the claims 1 to 3, wherein said disease is obsessive-compulsive disorder.

7. A peptide hormone for use according to any of the claims 1 to 3, wherein said disease is attention deficiency hyperactivity syndrome.

8. A peptide hormone for use according to any of the claims 1 to 3, wherein said disease is mania.

9. A peptide hormone for use according to any of the claims 1 to 3, wherein said disease is bipolar disorder.

10. A peptide hormone for use according to any of the claims 1 to 3, wherein said disease is a hyperactivity disorder manifested by a disease selected from the group consisting of attention deficiency hyperactivity syndrome, mania and bipolar disorder.

11. A pharmaceutical composition comprising the peptide hormone according to any of the claims 1 to 3 and at least one excipient, for use in treatment of a disease selected from schizophrenia, psychosis, obsessive-compulsive disorder, attention deficiency hyperactivity syndrome, mania and bipolar disorder.

## Patentansprüche

1. Peptidhormon der AKH/RPCH-Peptidfamilie zur Verwendung bei der Behandlung einer Erkrankung, die aus Schizophrenie, Psychose, Zwangsstörung, Aufmerksamkeitsdefizit-Hyperaktivitätsstörung, Manie und bipolarer Störung ausgewählt ist, wobei das Peptid die folgende Aminosäuresequenz aufweist:
**Xaa¹** - **Xaa²** - **Xaa³**- **Xaa⁴** - **Xaa⁵** - **Xaa⁶** - **Xaa⁷** - **Xaa⁸** - **X** - **NH₂**
wobei:
Xaa¹ ein Pyroglutamat-Rest ist,
der Xaa²-Rest aus einer Gruppe ausgewählt ist, die aus den Aminosäuren Leu, Val, Ile, Phe, Tyr besteht,
der Xaa³-Rest aus einer der Aminosäuren Asn, Thr ausgewählt ist,
der Xaa⁴-Rest aus einer der Aminosäuren Phe, Tyr ausgewählt ist,
der Xaa⁵-Rest aus einer der Aminosäuren Thr, Ser ausgewählt ist,
der Xaa⁶-Rest aus einer der Aminosäuren Pro, Ser, Thr, Ala ausgewählt ist,
der Xaa⁷-Rest aus einer der Aminosäuren Asn, Gly, Ser, Asp, Trp, Val ausgewählt ist,
der Xaa⁸-Rest als die Aminosäure Trp ausgewählt ist,
und der Rest X zwischen 0 bis 2 Aminosäurereste aufweist, vorzugsweise 0 (Aminosäurereste) aufweist, falls 1 bis 2 Aminosäuren vorhanden sind, steht ein Gly-Rest für die erste Aminosäure und einer der Reste Gly, Thr, Asn, Ser, Tyr, Trp für die zweite Aminosäure.

2. Peptidhormon zur Verwendung gemäß Anspruch 1, wobei das Peptidhormon die folgende Aminosäuresequenz aufweist:
**pGlu** - **Val** - **Asn** - **Phe** - **Xaa⁵** - **Pro** - **Ser** - **Trp** - **NH₂** wobei:
der Xaa⁵-Rest aus den Aminosäuren Ser und Thr ausgewählt ist.

3. Peptidhormon zur Verwendung gemäß Anspruch 1, wobei das Peptidhormon die folgende Aminosäuresequenz aufweist:
**pGlu** - **Leu** - **Thr** - **Phe** - **Ser** - **Pro** - **Asp** - **Trp** - **NH₂**

4. Peptidhormon zur Verwendung gemäß einem der Ansprüche 1 bis 3, wobei die Erkrankung Schizophrenie ist.

5. Peptidhormon zur Verwendung gemäß einem der Ansprüche 1 bis 3, wobei die Erkrankung eine Psychose ist.

6. Peptidhormon zur Verwendung gemäß einem der Ansprüche 1 bis 3, wobei die Erkrankung eine Zwangsstörung ist.

7. Peptidhormon zur Verwendung gemäß einem der Ansprüche 1 bis 3, wobei die Erkrankung Aufmerksamkeitsdefizit-Hyperaktivitätsstörung ist.

8. Peptidhormon zur Verwendung gemäß einem der Ansprüche 1 bis 3, wobei die Erkrankung Manie ist.

9. Peptidhormon zur Verwendung gemäß einem der Ansprüche 1 bis 3, wobei die Erkrankung eine bipolare Störung ist.

10. Peptidhormon zur Verwendung gemäß einem der Ansprüche 1 bis 3, wobei die Erkrankung eine Hyperaktivitätsstörung ist, die sich durch eine Erkrankung manifestiert, die aus der aus Aufmerksamkeitsdefizit-Hyperaktivitätsstörung, Manie und bipolarer Störung bestehenden Gruppe ausgewählt ist.

11. Pharmazeutische Zusammensetzung, die das Peptidhormon gemäß einem der Ansprüche 1 bis 3 und mindestens einen Hilfsstoff umfasst, zur Verwendung bei der Behandlung einer Erkrankung, die aus Schizophrenie, Psychose, Zwangsstörung, Aufmerksamkeitsdefizit-Hyperaktivitätsstörung, Manie und bipolarer Störung ausgewählt ist.

## Revendications

1. Une hormone peptidique de la famille des peptides AKH / RPCH destinée à être utilisée dans le traitement d'une maladie choisie parmi la schizophrénie, la psychose, le trouble obsessionnel-compulsif, le syndrome du déficit de l'attention avec hyperactivité, la manie et le trouble bipolaire; dans lequel ledit peptide présente la séquence d'acides aminés suivante:
**Xaa¹** - **Xaa²** - **Xaa³ - Xaa⁴** - **Xaa⁵** - **Xaa⁶ - Xaa⁷** - **Xaa⁸** - **X** - **NH₂**
où ;
Xaa¹ est un résidu pyroglutamate,
le résidu Xaa² est choisi parmi le groupe constitué des acides aminés Leu, Val, Ile, Phe, Tyr,
le résidu Xaa³ est choisi parmi l'un des acides aminés Asn, Thr,
le résidu Xaa⁴ est choisi parmi l'un des acides aminés Phe, Tyr,
le résidu Xaa⁵ est choisi parmi l'un des acides aminés Thr, Ser,
le résidu Xaa⁶ est choisi parmi l'un des acides aminés Pro, Ser, Thr, Ala,
le résidu Xaa⁷ est choisi parmi l'un des acides aminés Asn, Gly, Ser, Asp, Trp, Val,
le résidu Xaa⁸ est choisi en tant qu'acide aminé Trp,
et, le résidu X est compris entre 0 et 2 résidus d'acide aminé; il est de préference 0 dans le cas où il y a 1-2 acides aminés, il s'agit du résidu Gly pour le premier et de l'un des résidus Gly, Thr, Asn, Ser, Tyr, Trp pour le second.

2. Une hormone peptidique destinée a etre utilisée selon la revendication 1, dans lequel ladite hormone peptidique présent la séquence d'acides aminés suivante :
**pGlu-Val-Asn-Phe-Xaa⁵-Pro-Ser-Trp-NH₂**
où ; le résidu Xaa⁵ est choisi parmi des acides aminés Ser et Thr.

3. Une hormone peptidique destinée a etre utilisée selon la revendication 1, dans lequel ladite hormone peptidique présent la séquence d'acides aminés suivante :
**pGlu-Leu-Thr-Phe-Ser-Pro-Asp-Trp-NH₂**

4. Une hormone peptidique destinée a etre utilisée selon l'une quelconque des revendications 1 à 3, dans lequel ladite maladie est la schizophrénie.

5. Une hormone peptidique destinée a etre utilisée selon l'une quelconque des revendications 1 à 3, dans lequel ladite maladie est la psychose.

6. Une hormone peptidique destinée a etre utilisée selon l'une quelconque des revendications 1 à 3, dans lequel ladite maladie est le trouble obsessionnel-compulsif.

7. Une hormone peptidique destinée a etre utilisée selon l'une quelconque des revendications 1 à 3, dans lequel ladite maladie est le syndrome du déficit de l'attention avec hyperactivité.

8. Une hormone peptidique destinée a etre utilisée selon l'une quelconque des revendications 1 à 3, dans lequel ladite maladie est la manie.

9. Une hormone peptidique destinée a etre utilisée selon l'une quelconque des revendications 1 à 3, dans lequel ladite maladie est le trouble bipolaire.

10. Une hormone peptidique destinée a etre utilisée selon l'une quelconque des revendications 1 à 3, dans lequel ladite maladie est un trouble d'hyperactivité manifestant une maladie choisie parmi le groupe comprenant le syndrome du déficit de l'attention avec hyperactivité, la manie et le trouble bipolaire.

11. Composition pharmaceutique comprenant l'hormone peptidique selon l'une quelconque des revendications 1 à 3 et au moins un excipient, destinée à être utilisée dans le traitement d'une maladie choisie parmi la schizophrénie, la psychose, le trouble obsessionnel-compulsif, le syndrome du déficit de l'attention avec hyperactivité, la manie et le trouble bipolaire.
